(19) Europäisches Patentamt
European Patent Office
Office européen des brevets

(11) Veröffentlichungsnummer: **0 046 139**
**B1**

(12) **EUROPÄISCHE PATENTSCHRIFT**

(45) Veröffentlichungstag der Patentschrift: **18.07.84**

(51) Int. Cl.³: **C 23 F 11/14**

(21) Anmeldenummer: **81810317.8**

(22) Anmeldetag: **07.08.81**

(54) **Verwendung von Triazincarbonsäuren als Korrosionsinhibitoren für wässrige Systeme.**

(30) Priorität: **13.08.80 GB 8026311**

(43) Veröffentlichungstag der Anmeldung: **17.02.82 Patentblatt 82/7**

(45) Bekanntmachung des Hinweises auf die Patenterteilung: **18.07.84 Patentblatt 84/29**

(84) Benannte Vertragsstaaten: **DE FR GB IT**

(56) Entgegenhaltungen:
**FR - A - 2 390 440**
**US - A - 2 485 309**
**US - A - 3 165 515**
**US - A - 3 236 846**

(73) Patentinhaber: **CIBA-GEIGY AG, Postfach, CH-4002 Basel (CH)**

(72) Erfinder: **Clark, David Ronald, Dr., 16 Ennerdale Drive, Sale Cheshire (GB)**

**EP 0 046 139 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

**Beschreibung**

Die vorliegende Erfindung betrifft eine Methode zur Inhibierung der Korrosion von Eisen oder eisenhaltigen Metallen in Kontakt mit wäßrigen Systemen durch Zusatz von bestimmten Triazincarbonsäuren oder deren wasserlöslichen Salzen als Korrosionsinhibitor sowie die so erhaltenen antikorrosiven wäßrigen Systeme.

In neuerer Zeit ist viel technischer Aufwand geleistet worden, um die Probleme zu lösen, die mit der Korrosion von Metallen in Kontakt mit Wasserkreisläufen entstehen. Verschiedene korrosionsinhibierende Gemische wurden vorgeschlagen, beispielsweise die in der GB-A-1 374 270 beschriebenen Gemische von Polymaleinsäuren mit Zinksalzen oder die in der US-A- 3 133 028 beschriebenen Gemische von Thiocyanaten oder Thioharnstoffen mit Chromaten. Diese bekannten Gemische haben gewisse Nachteile. So belasten z. B. Schwermetalle wie Chrom oder Zink die Abwässer auf Grund ihrer Toxizität, und auf Grund der verschärften Umweltschutzbestimmungen hat man nach Alternativen gesucht. Andere, schwermetallfreie Korrosionsinhibitoren sind z. B. die in der GB-A-1 092 044 beschriebenen synergistischen Gemische von Aminophosphonsäuren mit Nitriten. Diese haben den Nachteil, daß im Laufe des Gebrauches das Nitrit zu Nitrat oxydiert wird, wodurch die Wirkung nachläßt und Algenwachstum gefördert wird. Außerdem kann Nitrit mit Aminen unter Bildung toxischer N-Nitrosoverbindungen reagieren.

Auch die Methylolmelamin-Kondensationsprodukte der US-A-2 485 309 und die ungesättigten Triazinderivate der US-A-3 165 515 genügen nicht vollends den hohen Anforderungen, welche heute an ein Korrosionsschutzmittel gestellt werden.

Es wurde nun gefunden, daß bestimmte Triazincarbonsäuren und ihre wasserlöslichen Salze ausgezeichnete Korrosionsinhibitoren in wäßrigen Systemen sind, wie z. B. in Wasserkreisläufen, in wäßrigen Maschinenflüssigkeiten, Gefrierschutzflüssigkeiten, hydraulischen Flüssigkeiten oder wäßrigen Anstrichmitteln.

Die vorliegende Erfindung betrifft die Verwendung von Verbindungen der Formel I

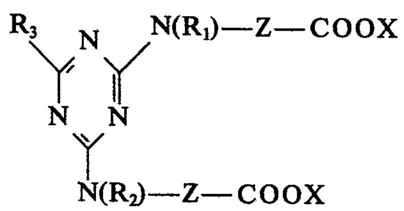

(I)

worin Z eine $C_1-C_{11}$-Alkylengruppe ist, X Wasserstoff, ein Alkalimetall, Mono-, Di- oder Triethanolammonium ist, $R_1$ und $R_2$ Wasserstoff oder Methyl sind und $R_3$ eine Gruppe $-NR_4R_5$ ist, worin $R_4$ $-Z-COOX$ oder $C_1-C_{12}$-Alkyl und $R_5$ Wasserstoff oder $C_1-C_{12}$-Alkyl sind, als Korrosionsinhibitoren für wäßrige Systeme, die in Kontakt mit Eisen oder eisenhaltigen Metallen stehen.

Ist Z Alkylen mit 1—11 C-Atomen, so handelt es sich z. B. um Methylen, 1,2-Ethylen, 1,2-Propylen, 1,3-Propylen, 1,4-Butylen, 1,1-Dimethyl-1,2-ethylen, 1,5-Pentylen, 1,6-Hexylen, 1,8-Octylen, 1,10-Decylen oder 1,11-Undecylen. Bevorzugt ist Z Alkylen mit 1—5 C-Atomen.

Wenn X ein Alkalimetall ist, so kann das Na, K oder Li sein.

Bevorzugt ist $R_3$ eine Gruppe $-NR_4R_5$, worin $R_4$ $-Z-COOX$ oder $C_1-C_8$-Alkyl und $R_5$ H, $CH_3$ oder $C_2H_5$ sind.

Beispiele für spezifische Verbindungen der Formel I sind die folgenden:

2,4,6-Tris(5′-carboxypentylamino)-1,3,5-triazin
2,4,6-Tris(carboxymethylamino)-1,3,5-triazin
2,4,6-Tris(3′-carboxypropylamino)-1,3,5-triazin
2,4,6-Tris(2′-carboxyethylamino)-1,3,5-triazin
2,4,6-Tris(4′-carboxybutylamino)-1,3,5-triazin
2,4,6-Tris(11′-carboxyundecylamino)-1,3,5-triazin
2,4,6-Tris(5′-carboxypentyl-N-methylamino)-1,3,5-triazin
2,4,6-Tris(carboxymethyl-N-methylamino)-1,3,5-triazin
2,4,6-Tris(3′-carboxypropyl-N-methylamino)-1,3,5-triazin
2,4-Bis(5′-carboxypentylamino)-6-ethylamino-1,3,5-triazin
2,4-Bis(5′-carboxypentylamino)-6-n-octylamino-1,3,5-triazin
2,4-Bis(5′-carboxypentylamino)-6-diethylamino-1,3,5-triazin
2,4-Bis(2′-carboxyethylamino)-6-n-octylamino-1,3,5-triazin
2,4-Bis(2′-carboxyethylamino)-6-diethylamino-1,3,5-triazin
2,4-Bis(2′-carboxyethylamino)-6-butylamino-1,3,5-triazin
2,4-Bis(2′-carboxyethylamino)-6-ethylamino-1,3,5-triazin
2,4-Bis(3′-carboxypropylamino)-6-ethylamino-1,3,5-triazin
2,4-Bis(3′-carboxypropylamino)-6-n-octylamino-1,3,5-triazin

2,4-Bis(3'-carboxypropylamino)-6-n-propylamino-1,3,5-triazin
2,4-Bis(carboxymethylamino)-6-n-octylamino-1,3,5-triazin
2,4-Bis(carboxymethylamino)-6-dodecylamino-1,3,5-triazin
2,4-Bis(5'-carboxypentyl-N-methylamino)-6-ethylamino-1,3,5-triazin
2,4-Bis(3'-carboxypropyl-N-methylamino)-6-n-octylamino-1,3,5-triazin
2,4-Bis(carboxymethyl-N-methylamino)-6-n-octylamino-1,3,5-triazin
2,4-Bis(2'-carboxyethyl-N-methylamino)-6-n-octylamino-1,3,5-triazin
2,4-Bis(5'-carboxypentylamino)-6-(2''-carboxyethylamino)-1,3,5-triazin
2,4-Bis(5'-carboxypentylamino)-6-(carboxymethylamino)-1,3,5-triazin
2,4-Bis(3'-carboxypropylamino)-6-(2''-carboxyethylamino)-1,3,5-triazin
2,4-Bis(2'-carboxyethylamino)-6-(5''-carboxypentylamino)-1,3,5-triazin
2,4-Bis(3'-carboxypropylamino)-6-(5''-carboxypentylamino)-1,3,5-triazin

Die Verbindungen der Formel I sind nicht neu. Solche Verbindungen und ihre Herstellung sind beispielsweise beschrieben in Zhurnal Analiticheskoi Khimii 15, 419—423 (1960), in der DE-OS 1 935 010, in der DE-OS 2 819 796, in der US-PS 3 697 520 und in J. Prakt. Chemie 23, 173—85 (1963). In keiner dieser Literaturstellen wird jedoch die Verwendung der Verbindungen der Formel I als Korrosionsinhibitoren für Eisen in wäßrigen Systemen beschrieben oder nahegelegt.

Die Verbindungen der Formel I werden vorzugsweise in einer Menge von 0,001 bis 5 Gew.-%, bezogen auf das wäßrige System, verwendet.

Wie bereits vorhin erwähnt, kann es sich bei den wäßrigen Systemen um Wasserkreisläufe handeln, beispielsweise Kühlwasserkreisläufe, es kann sich um wäßrige Maschinenflüssigkeiten handeln, wie sie beispielsweise als Kühlflüssigkeiten beim Bohren, Mahlen, Fräsen, Drehen, Schneiden, Sägen, Schleifen, Gewindeschneiden oder beim Walzen oder Ziehen von Metallen verwendet werden, es kann sich um Gefrierschutzmittel oder hydraulische Flüssigkeiten auf Glykol-Wasser-Basis handeln, und es kann sich um wäßrige Anstrichstoffe handeln, wie z. B. Dispersionsfarben oder wäßrige Pulverlacke.

Die Verbindungen der Formel I können in den wäßrigen Systemen als alleiniger Zusatz verwendet werden oder in Kombination mit anderen Zusätzen. Beispiele für solche Co-Additive in Wasserkreisläufen sind bekannte Korrosionsinhibitoren wie Phosphonate, Phosphonocarbonsäuren oder Phosphinocarbonsäuren, N-Acylsarkosine, Imidazoline, Triethanolamin, Fettamine oder Polycarbonsäuren. Diese können Kupfer-Passivatoren sein, wie z. B. wasserlösliche Benztriazole, Methylen-bis-benztriazole oder 2-Mercaptobenzthiazole. Diese können Dispersionsmittel und Trägerstoffe sein, wie z. B. Poly(meth)acrylsäure und seine Salze, hydrolysiertes Polyacrylnitril, Polyacrylamid und dessen Copolymere, Ligninsulfonsäure und deren Salze, Stärke und Stärkederivate, Cellulose, Alkylphosphonsäuren, 1-Aminoalkyl-1,1-diphosphonsäuren und ihre Salze, Polymaleinsäuren und andere Polycarbonsäuren oder Alkaliphosphate.

Weitere Co-Additive können Fällungsmittel sein, wie z. B. Alkaliphosphate oder Alkalicarbonate, Sauerstoffabfänger, wie z. B. Alkalisulfate oder Hydrazin, Komplexierungsmittel, wie z. B. Nitrilotriessigsäure oder Ethylendiamin-tetraessigsäure und deren Salze, oder schaumverhütende Mittel, wie z. B. Distearylsebacinsäurediamid, Disterayladipinsäurediamid oder Ethylenoxid- oder Propylenoxid-Kondensationsprodukte solcher Amide, sowie Fettalkohole und deren Ethylenoxid-Kondensationsprodukte.

Wäßrige Systeme, die als Maschinenflüssigkeiten verwendet werden, können ein wasserverdünnbares Schneid- oder Schleiföl sein, wie beispielsweise

a) wäßrige Konzentrate eines oder mehrerer Korrosionsinhibitoren mit oder ohne einen Antiverschleißzusatz, die dann in einer Verdünnung von 1 : 50 bis 1 : 100 als Schleifflüssigkeit verwendet werden können,
b) Polyglykole, die Biocide, Korrosionsinhibitoren und Antiverschleißmittel enthalten und die als Schneidflüssigkeit in einer Verdünnung von 1 : 20 bis 1 : 40 und als Schleifflüssigkeit in einer Verdünnung von 1 : 60 bis 1 : 80 verwendet werden können,
c) halbsynthetische Schneidöle auf ähnlicher Basis wie b), jedoch zusätzlich 10—25% eines Öls enthaltend sowie genügend Emulgator, um die Flüssigkeit beim Verdünnen transparent zu halten,
d) emulgierbare Mineralöl-Konzentrate, die außer dem Emulgator auch Korrosionsinhibitoren, Antiverschleißmittel, Biocide, Antischaummittel enthalten können und die üblicherweise im Verhältnis 1 : 20 bis 1 : 50 mit Wasser verdünnt werden zu einer opaken Emulsion,
e) Produkte ähnlich d), jedoch weniger Öl und mehr Emulgator enthaltend, die bei einer Verdünnung von 1 : 50 bis 1 : 100 durchscheinende Emulsionen ergeben.

Auch für diese Maschinenflüssigkeiten sowie Gefrierschutzmittel oder Hydraulikflüssigkeiten können die Verbindungen der Formel I entweder allein oder in Kombination mit anderen Zusätzen verwendet werden. Darin können z. B. andere Korrosionsinhibitoren sein, wie

a) organische Säuren, deren Salze und Ester, z. B. Benzoesäure, p-tert.Butylbenzoesäure, Dinatriumsebacat, Triethanolamin-laurat, Isononansäure, das Triethanolaminsalz von p-Toluolsulfonamidocapronsäure, Natrium-N-lauroylsarcosinat oder Nonylphenoxyessigsäure;

b) stickstoffhaltige Substanzen, wie z. B. Fettsäurealkanolamide, Imidazoline, Oxazoline, Triazole oder anorganische Nitrite oder Nitrate;

c) phosphorhaltige Substanzen, beispielsweise Aminphosphate, Phosphonsäuren oder anorganische Phosphate, wie z. B. $NaH_2PO_4$;

d) schwefelhaltige Substanzen, beispielsweise Salze von Petroleumsulfonaten, oder heterocyclische Verbindungen wie Natrium-mercaptobenzthiazol.

Beispiele von Extremdruck-Antiverschleißzusätzen, die in funktionellen Flüssigkeiten enthalten sein können, sind Schwefel, Phosphor oder Halogen enthaltende Substanzen, z. B. sulfoniertes Spermöl, sulfurierte Fette, Tritolylphosphat, Chlorparaffine oder ethoxylierte Phosphatester.

Die folgenden Beispiele 1—15 illustrieren die Herstellung von Verbindungen der Formel I, während die Beispiele 16—38 ihre Verwendung in verschiedenen wäßrigen Systemen zeigen. Darin beziehen sich Teile und Prozentangaben auf Gewichtsteile. Die Temperaturen beziehen sich auf ° Celsius.

## Beispiel 1

### Herstellung von 2,4,6-Tris(5'-carboxypentylamino)-1,3,5-triazin

In eine Suspension von 18,5 Teilen Cyanursäurechlorid in 150 Teilen Wasser von 0° wird ein Drittel einer Lösung von 49 Teilen 6-Aminohexansäure-Natrium in 70 Teilen Wasser innerhalb $1^1/_2$ Std. zugetropft, wobei die Temperatur unter 5° gehalten wird. Dann wird der Rest der Lösung zugegeben und das Reaktionsgemisch erst 2 Std. bei Raumtemperatur, dann 3 Std. unter Rückfluß gerührt. Während dieser Zeit wird der pH-Wert durch Zugabe von 25%iger NaOH laufend auf 10 bis 11 gehalten.

Das Reaktionsgemisch wird auf 50° abgekühlt, filtriert, das Filtrat mit 1000 Teilen Wasser verdünnt und mit konzentrierter Salzsäure auf einen pH von 4—4,5 angesäuert, wobei sich ein kristalliner Niederschlag bildet. Nachdem das Reaktionsgemisch auf Raumtemperatur abgekühlt ist, wird der Niederschlag abfiltriert, mit kaltem Wasser gewaschen und im Vakuum getrocknet. Man erhält 35 Teile 2,4,6-Tris(5'-carboxypentylamino)-1,3,5-triazin, entsprechend einer Ausbeute von 75%. Nach Umkristallisation aus wäßriger Essigsäure schmilzt die Substanz bei 177° C.

Analyse ($C_{21}H_{36}N_6O_6$)

| | | | |
|---|---|---|---|
| ber.: | C 53,85 | H 7,69 | N 17,95%; |
| gef.: | C 54,07 | H 7,68 | N 17,26%. |

Analog dazu wurden die in Tabelle I angeführten Verbindungen der Beispiele 2—7 hergestellt.

## Beispiel 8

### Herstellung von 2-n-Octylamino-4,6-bis(2'-carboxyethylamino)-1,3,5-triazin

18,5 Teile Cyanursäurechlorid werden mit 200 Teilen Aceton behandelt und die opake Lösung in 300 Teile Eiswasser eingerührt. Zu dieser Suspension von frisch gefälltem Cyanurchlorid tropft man bei 0 bis 5° 28 Teile n-Octylamin innerhalb einer Stunde zu und hält dabei durch Zugabe von 25%iger NaOH den pH-Wert auf 5—6.

Dann gibt man eine Lösung von 42,4 Teilen Natriumsalz des $\beta$-Alanin in 60 Teilen Wasser zu und läßt die Reaktionsmischung durch 2stündiges Rühren auf Raumtemperatur kommen.

Dann erwärmt man langsam auf 95° unter ständigem Abdestillieren des Aceton und hält dabei den pH-Wert durch Zugabe von weiterer Natronlauge auf 10—11. Die Reaktion wird schließlich nach 6 Std. Erwärmen auf 95—100° bei pH 10—11 vervollständigt.

Nach Auskühlen auf 30° wird die Lösung mit konzentrierter Salzsäure auf einen pH von 4—4,5 angesäuert, wobei ein weißer Niederschlag ausfällt. Dieser wird abfiltriert, mit Wasser gewaschen und im Vakuum getrocknet. Man erhält 26,4 Teile 2-n-Octylamino-4,6-bis(2'-carboxyethylamino)-1,3,5-triazin, entsprechend einer Ausbeute von 69%. Nach Umkristallisation aus Wasser schmilzt die Substanz bei 138—140°.

Analyse ($C_{11}H_{50}N_6O_4$)

| | | | |
|---|---|---|---|
| ber.: | C 53,40 | H 7,85 | N 21,99%; |
| gef.: | C 52,91 | H 7,97 | N 21,65%. |

In analoger Weise wurden die in Tabelle I aufgeführten Verbindungen der Beispiele 9—15 hergestellt.

Tabelle I
Hergestellte Verbindungen der Formel

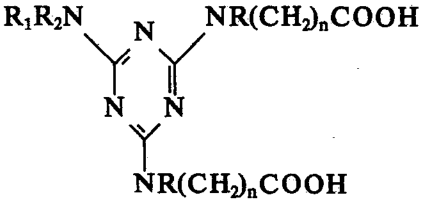

| Beispiel | R | $R_1$ | $R_2$ | n | Ausbeute (%) | Fp. (°C) | Analyse (%) | C | H | N |
|---|---|---|---|---|---|---|---|---|---|---|
| 2 | H | $(CH_2)_3COOH$ | H | 3 | 67 | 205,7 | $C_{15}H_{24}N_6O_6 \cdot H_2O$ | | | |
| | | | | | | | ber. | 44,77 | 6,47 | 20,90 |
| | | | | | | | gef. | 44,60 | 6,33 | 20,48 |
| 3 | H | $(CH_2)_2COOH$ | H | 2 | 73 | > 230 | $C_{12}H_{18}N_6O_6$ | | | |
| | | | | | | | ber. | 42,11 | 5,26 | 24,56 |
| | | | | | | | gef. | 42,24 | 5,27 | 24,24 |
| 4 | H | $CH_2COOH$ | H | 1 | 92 | > 230 | $C_9H_{12}N_6O_6$ | | | |
| | | | | | | | ber. | 36,00 | 4,00 | 28,00 |
| | | | | | | | gef. | 35,13 | 3,95 | 27,44 |
| 5 | $CH_3$ | $(CH_2)_5COOH$ | $CH_3$ | 5 | 45 | 109—11 | $C_{24}H_{42}N_6O_6$ | | | |
| | | | | | | | ber. | 56,47 | 8,24 | 16,47 |
| | | | | | | | gef. | 56,23 | 7,84 | 16,97 |
| 6 | $CH_3$ | $(CH_2)_3COOH$ | $CH_3$ | 3 | 61 | 146—7 | $C_{18}H_{30}N_6O_6$ | | | |
| | | | | | | | ber. | 50,70 | 7,04 | 19,72 |
| | | | | | | | gef. | 51,15 | 7,10 | 19,68 |
| 7 | $CH_3$ | $CH_2COOH$ | $CH_3$ | 1 | 65 | 225—6 | $C_{12}H_{18}N_6O_6$ | | | |
| | | | | | | | ber. | 42,11 | 5,26 | 24,56 |
| | | | | | | | gef. | 41,80 | 5,11 | 24,10 |
| 9 | H | n-$C_8H_{17}$ | H | 3 | 78 | 174—6 | $C_{19}H_{34}N_6O_4$ | | | |
| | | | | | | | ber. | 55,59 | 8,35 | 20,48 |
| | | | | | | | gef. | 55,46 | 8,51 | 20,54 |
| 10 | H | n-$C_8H_{17}$ | H | 5 | 69 | 119—21 | $C_{23}H_{42}N_6O_4$ | | | |
| | | | | | | | ber. | 59,23 | 9,01 | 18,03 |
| | | | | | | | gef. | 59,06 | 8,97 | 18,05 |
| 11 | H | $C_2H_5$ | H | 2 | 73 | 229—31 | $C_{11}H_{18}N_6O_4$ | | | |
| | | | | | | | ber. | 44,30 | 6,04 | 28,18 |
| | | | | | | | gef. | 43,63 | 6,01 | 27,66 |
| 12 | H | $C_2H_5$ | $CH_3$ | 2 | 73 | 208—10 | $C_{13}H_{22}N_6O_4$ | | | |
| | | | | | | | ber. | 47,85 | 6,75 | 25,77 |
| | | | | | | | gef. | 46,85 | 6,84 | 24,25 |
| 13 | H | $C_2H_5$ | H | 3 | 63 | 192—4 | $C_{13}H_{22}N_6O_4$ | | | |
| | | | | | | | ber. | 47,85 | 6,75 | 25,77 |
| | | | | | | | gef. | 46,72 | 7,09 | 25,39 |
| 14 | H | $C_2H_5$ | H | 5 | 79 | 150—2 | $C_{17}H_{30}N_6O_4$ | | | |
| | | | | | | | ber. | 53,40 | 7,85 | 21,99 |
| | | | | | | | gef. | 52,19 | 8,08 | 21,00 |
| 15 | H | $C_2H_5$ | $C_2H_5$ | 5 | 66 | 149—51 | $C_{19}H_{34}N_6O_4$ | | | |
| | | | | | | | ber. | 55,61 | 8,29 | 20,49 |
| | | | | | | | gef. | 54,61 | 7,70 | 19,84 |

Beispiele 16—30

Korrosionsschutzwirkung in belüftetem Wasser

Die korrosionsinhibierende Wirksamkeit der Verbindungen der Beispiele 1—15 wird in einem Test in Flaschen mit belüftetem Wasser gezeigt, wobei folgende Arten von Wasser verwendet werden:

A) Künstlich korrosiv gemachtes Wasser von pH 7,5 und einer Härte von 30 ppm $CaCO_3$,
B) angesäuertes Wasser von pH 6,5 und Härte von 200 ppm $CaCO_3$,
C) mittels basischem Ionenaustauscher behandeltes Wasser von pH 7,5 und Härte 0.

Bleche aus kohlenstoffarmem Stahl von 5×2,5×0,1 cm werden mit Bimsstein gescheuert, für 1 Minute in verdünnte Salzsäure getaucht, mit Wasser gespült, getrocknet und gewogen.

Die einzelnen Korrosionsinhibitoren werden als Natriumsalz in den verschiedenen Wasserproben gelöst, wobei eine Konzentration von 100 ppm eingestellt wird. In jede dieser Lösungen, die in Flaschen gefüllt werden, wird ein Stahlblech gelegt, und die Flaschen werden auf 40°C thermostatisiert. Durch die Testlösungen wird Luft durchgeleitet in einer Menge von 500 ml/Minute. Das mit dem Luftstrom verdunstende Wasser wird ständig durch destilliertes Wasser ersetzt, so daß das Volumen gleich bleibt.

Nach 48 Std. wird das Stahlblech aus der Lösung genommen, mit Bimsstein gescheuert, für 1 Minute in verdünnte Salzsäure, die 1% Hexamethylentetramin enthält, getaucht, dann mit Wasser gespült, getrocknet und durch Rückwägung der Gewichtsverlust der Bleche bestimmt. In jeder Testserie läuft eine Blindprobe ohne Korrosionsinhibitor mit. Der »Korrosionsgrad« ist der Gewichtsverlust in mg pro $dm^2$ der Probenoberfläche pro Tag.

Anschaulicher ist die Angabe des »prozentuellen Korrosionsschutzes« (% KS), der sich wie folgt berechnet:

$$\%KS = \frac{\text{Korrosionsgrad der Blindprobe} - \text{Korrosionsgrad der Probe}}{\text{Korrosionsgrad der Blindprobe}} \times 100.$$

Tabelle II

| Beispiel | Korrosions-inhibitor aus Beispiel*) | % KS im Wasser A | B | C |
|---|---|---|---|---|
| 16 | 1 | 95 | 95 | 93 |
| 17 | 2 | 100 | 96 | 98 |
| 18 | 3 | 99 | 96 | 97 |
| 19 | 4 | (40) | (23) | 92 |
| 20 | 5 | 55 | 86 | 96 |
| 21 | 6 | 86 | (28) | 97 |
| 22 | 7 | 76 | (30) | 74 |
| 23 | 8 | 63 | 89 | 100 |
| 24 | 9 | 60 | (15) | 98 |
| 25 | 10 | 96 | 100 | 100 |
| 26 | 11 | 90 | 86 | 96 |
| 27 | 12 | — | — | 100 |
| 28 | 13 | 86 | 96 | 97 |
| 29 | 14 | 70 | 100 | 100 |
| 30 | 15 | 97 | 95 | 100 |

*) Die Verbindungen wurden mit NaOH neutralisiert.

Die Resultate zeigen, daß alle Verbindungen in den verschiedenen Wasserproben eine ausgezeichnete Korrosionsschutzwirkung haben.

## Beispiele 31—33

### Korrosionsschutzwirkung in einer laboratoriumsmäßigen Wärmeaustauscher-Modellvorrichtung

Die Vorrichtung stellt einen geschlossenen Wasserkreislauf dar, bestehend aus einem 20-l-Behälter, einem 1-l-Behälter, einer Pumpe, einem Durchflußmesser, einer Probenkammer, einem Wärmetauscher und einem Kühler.

Korrosives Gebrauchswasser wird im 20-l-Behälter mit 5 l/min Druckluft durch eine Sinterplatte begast und von dort in den 1-l-Behälter gepumpt. Von dort fließt das Wasser durch den Durchflußmesser in die gläserne Probenkammer, in der sich Metallbleche von 2,5 × 2,5 cm auf einer Halterung aus Acrylglas befinden.

Das Wasser fließt dann durch den Wärmeaustauscher, der aus einem Stahlrohr von 1,58 cm lichter Weite besteht. Das Rohr hat Enden aus Kupfer und ist umwickelt von einer 960-Watt-Heizspule. Nach dem Wärmeaustauscher fließt das Wasser durch den Kühler zurück in den 20-l-Behälter.

Im stationären Zustand kreist das Wasser mit 4,55 l/min durch die Vorrichtung. Dabei hat es im Wärmeaustauscher eine Geschwindigkeit von 0,46 m/sec und eine Reynoldszahl von 8500. Die Heizspule gibt dem Wärmeaustauscher eine Außentemperatur von 60°. Das Wasser verläßt den Wärmeaustauscher mit 45°. Der Kühler wird so betrieben, daß es das Wasser auf 40° herabkühlt, bevor es einen neuen Kreislauf beginnt.

Die Proben bestehen aus Blechen aus kohlenstoffarmem Stahl, Gußeisen, Kupfer, Messing und Aluminium. Sie werden vor dem Test mit Scheuerpulver gereinigt und dann in ein Säurebad getaucht, und zwar das Stahl-, Eisen-, Kupfer- und Messingblech für 1 Minute in 1 : 1 verdünnte konzentrierte Salzsäure von Raumtemperatur, die Aluminiumbleche für 5 Minuten in 5%ige Phosphorsäure mit 2% Chromsäure bei 75°. Anschließend werden die Bleche mit Wasser gespült, getrocknet und gewogen. Dann werden sie auf die Acrylglas-Halterung montiert, wobei darauf geachtet wird, daß sie sich weder gegenseitig noch mit Metallschrauben der Halterung berühren. Das Rohr des Wärmeaustauschers wird so wie die Stahlblech-Probe vorbehandelt und gewogen.

Dann wird die Vorrichtung zusammengebaut und durch Zirkulation von Salzsäure (1 : 1 verdünnt) gründlich gereinigt. Dann spült man mit frischem Leitungswasser etwa eine halbe Stunde und entleert die Vorrichtung. In einen der Behälter füllt man nun den Korrosionsinhibitor und füllt die Apparatur mit 22 l eines standardisierten Korrosionstest-Wassers.

Durch Einschalten der Pumpe und der Heizung wird der Kreislauf in Gang gebracht. Die Inhibitor-Konzentration und die Wassermenge in der Vorrichtung wird täglich kontrolliert. Nach 3 Tagen und nach weiteren 10 Tagen wird das Wärmeaustauschrohr ausgebaut und werden die Probenbleche entnommen, wie bei der Vorbehandlung gereinigt und zurückgewogen. In den ersten 3 Tagen des Testes werden 100 ppm Korrosionsinhibitor verwendet, im anschließenden 10-Tage-Test nur 25 ppm.

Tabelle III gibt die nach 13 Tagen erhaltenen Korrosionsgrade an, die sich aus dem gewogenen Gewichtsverlust ableiten.

Tabelle III

| Beispiel | Verwendeter Korrosionsinhibitor | Verwendetes Wasser | Korrosionsgrad in mg/dm/Tag Stahl | Al | Cu | Messing | Austauscherrohr |
|---|---|---|---|---|---|---|---|
| 31 | — | Manchester Wasser, pH 7,0 | 140,1 | 5,8 | 3,7 | 4,8 | 420,6 |
| | Kl-1*) | Manchester Wasser, pH 7,0 | 2,5 | 2,7 | 0,9 | 2,0 | 39,8 |
| 32 | — | über basischen Ionenaustauscher gereinigtes Wasser, pH 8,0 | 139,9 | 0,0 | 1,0 | 1,8 | 485,3 |
| | Kl-1*) | über basischen Ionenaustauscher gereinigtes Wasser, pH 8,0 | 6,4 | 4,0 | 0,9 | 0,5 | 11,2 |
| 33 | — | Macclesfield Wasser, pH 6,5 | 67,8 | 8,2 | 0,6 | 0,5 | 215,4 |
| | Kl-1*) | Macclesfield Wasser, pH 6,5 | 19,9 | 0,0 | 1,1 | 0,9 | 38,5 |

Kl-1 = Natriumsalz der Verbindung des Beispiels 1.

Beispiele 34—38

Korrosionsschutz in wäßrigen Schneidölen

Die Korrosionsinhibierung von wäßrigen Schneidölen gemäß vorliegender Erfindung wird nach der im folgenden geschilderten Methode getestet, die eine Modifikation der Testmethode 287 des Institute of Petroleum darstellt.

Eine 1%ige Lösung des Korrosionsinhibitors wird mit Triethanolamin auf einen pH von 9 neutralisiert. Diese Lösung wird weiter auf das 2-, 4-, 8- und 16fache Volumen verdünnt. Die Konzentration dieser Teillösungen an Inhibitor, berechnet auf die freie Triazincarbonsäure, beträgt also 0,5%, 0,25%, 0,12% und 0,06%.

Gußeisenspäne auf Filterpapier werden mit diesen Testlösungen übergossen und gemäß IP-Testmethode 287 die Anrostung des Papiers nach 2 Stunden visuell beurteilt. Dabei bedeuten:

0 — keine Rostflecken
T — ≤5 keine Flecken
M — ≤10% der Fläche rostig
S — >10% der Fläche rostig.

Die Tests wurden A) mit normalem (hartem) Wasser und B) mit entionisiertem Wasser gemacht. Außerdem wurde die Empfindlichkeit gegen hartes Wasser getestet. Hierbei bedeutet O keine Trübung. Die Ergebnisse sind in Tabelle IV aufgeführt.

Tabelle IV

| Beispiel | Verwendeter Inhibitor (Verbindung des Beispiels Nr. und Konzentration*) | Anrostung in A | B | Härteempfindlichkeit |
|---|---|---|---|---|
| 34 | 1 — 0,5% | 0 | 0 | 0 |
| | 0,25% | 0 | 0 | |
| | 0,12% | 0 | T—M | |
| | 0,06% | T—M | — | |
| 35 | 5 — 0,5% | 0 | 0 | 0 |
| | 0,25% | 0 | 0 | |
| | 0,12 | 0 | 0 | |
| | 0,06% | T | — | |
| 36 | 11 — 0,5% | 0 | 0 | 0 |
| | 0,25% | 0 | S | |
| | 0,12% | 0 | — | |
| | 0,06% | S | — | |
| 37 | 14 — 0,5% | 0 | 0 | 0 |
| | 0,25% | 0 | 0—T | |
| | 0,12% | 0 | S | |
| | 0,06% | 0 | — | |
| 38 | 15 — 0,5% | 0 | 0 | 0 |
| | 0,25% | 0 | 0 | |
| | 0,12% | T | M | |
| | 0,06% | T | — | |

*) Berechnet auf freie Triazincarbonsäure

**Patentansprüche**

1. Verwendung von Verbindungen der Formel I

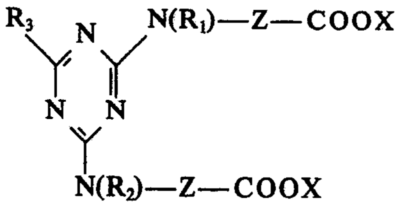

(I)

worin Z eine $C_1-C_{11}$-Alkylengruppe ist, X Wasserstoff, ein Alkalimetall, Mono-, Di- oder Triethanolammonium ist, $R_1$ und $R_2$ Wasserstoff oder Methyl sind und $R_3$ eine Gruppe $-NR_4R_5$ ist, worin $R_4$ $-Z-COOX$ oder $C_1-C_{12}$-Alkyl und $R_5$ Wasserstoff oder $C_1-C_{12}$-Alkyl sind als Korrosionsinhibitoren für wäßrige Systeme, die in Kontakt mit Eisen oder eisenhaltigen Metallen stehen.

2. Verwendung gemäß Anspruch 2, worin Z eine $C_1-C_5$-Alkylengruppe, $R_4$ $-Z-COOX$ oder $C_1-C_8$-Alkyl und $R_5$ Wasserstoff, Methyl oder Ethyl sind.

3. Wäßriges System, das außer dem Korrosionsinhibitor der im Anspruch 1 angegebenen Formel I noch einen anderen Korrosionsinhibitor enthält.

**Claims**

1. Use of compounds of the formula I

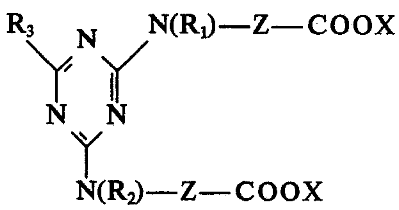

(I)

wherein Z is a $C_1-C_{11}$-alkylene group, X is hydrogen, an alkali metal, mono-, di- or triethanolammonium, $R_1$ and $R_2$ are hydrogen or methyl, and $R_3$ is a group $-NR_4R_5$, in which $R_4$ is $-Z-CCOOX$ or $C_1-C_{12}$-alkyl, and $R_5$ is hydrogen or $C_1-C_{12}$-alkyl, as corrosion inhibitors for aquwous systems which are in contact with iron or ferrous metals.

2. Use according to claim 2, wherein Z is a $C_1-C_5$-alkylene group, $R_4$ is $-Z-COOX$ or $C_1-C_8$-alkyl, and $R_5$ is hydrogen, methyl or ethyl.

3. Aqueous system which contains, besides the corrosion inhibitor of the formula I given in claim 1, a further corrosion inhibitor.

**Revendications**

1. Application de composés répondant à la formule I:

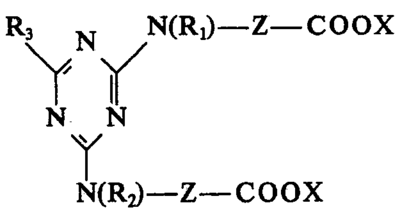

(I)

dans laquelle Z représente un radical alkylène en $C_1-C_{11}$, X représente l'hydrogène, un métal alcalin ou un radical mono-, di- ou triéthanolammonium, $R_1$ et $R_2$ représentent chacun l'hydrogène ou un radical méthyle et $R_3$ représente un radical $-NR_4R_5$ dans lequel $R_4$ désigne un radical $-Z-COOX$ ou un alkyle en $C_1-C_{12}$ et $R_5$ désigne l'hydrogène ou un alkyle en $C_1-C_{12}$, comme inhibiteurs de corrosion pour des systèmes aqeux qui entrent en contact avec du fer ou des métaux contenant du fer.

2. Application selon la revendication 2 caractérisée en ce que Z représente un radical alkylène en $C_1-C_5$, $R_4$ représente un radical $-Z-COOX$ ou un alkyle en $C_1-C_8$, et $R_5$ représente l'hydrogène ou un radical méthyle ou éthyle.

3. Système aqueux qui, en plus d'un inhibiteur de corrosion de formule I selon la revensication 1, contient un autre inhibiteur de corrosion.